# FASCICULE DE BREVET EUROPEEN

(11) **EP 4 155 717 B1**
(45) Date de publication et mention de la délivrance du brevet: **20.03.2024**
(21) Numéro de dépôt: 22197160.9
(22) Date de dépôt: 22.09.2022
(51) Int. Cl.: G01N 21/77, G01N 33/543, G02B 6/12, B82Y 15/00, B82Y 20/00

(54) **DISPOSITIF ET PROCÉDÉ DE DÉTECTION D'ESPÈCES CHIMIQUES OU BIOLOGIQUES**
VORRICHTUNG UND VERFAHREN ZUM NACHWEIS CHEMISCHER ODER BIOLOGISCHER SPEZIES
DEVICE AND METHOD FOR DETECTING CHEMICAL OR BIOLOGICAL SPECIES

(30) Priorité: 24.09.2021 FR 2110087
(43) Date de publication de la demande: 29.03.2023
(73) Titulaire: COMMISSARIAT À L'ÉNERGIE ATOMIQUE ET AUX ÉNERGIES ALTERNATIVES, 75015 Paris (FR)
(72) Inventeur: ELMI DAWALE, Houssein, 38054 Grenoble Cedex 09 (FR); BADETS, Franck, 38054 Grenoble Cedex 09 (FR); HENTZ, Sébastien, 38054 Grenoble Cedex 09 (FR); JOURDAN, Guillaume, 38054 Grenoble Cedex 09 (FR); SANSA PERNA, Marc, 38054 Grenoble Cedex 09 (FR); SIBEUD, Loïc, 38054 Grenoble Cedex 09 (FR)
(74) Mandataire: Atout PI Laplace

(56) Documents cités:
- EP-A1- 2 515 099
- WO-A1-01/63336
- US-A1- 2012 182 552
- US-A1- 2014 003 761
- US-A1- 2016 246 000
- US-B1- 9 612 458

## Description

### Art antérieur

La présente invention concerne généralement le domaine de l'instrumentation de détection d'espèces chimiques ou biologiques, et en particulier un dispositif et un procédé de détection d'espèces. L'invention concerne également un procédé de fabrication d'un tel dispositif.

La spectrométrie de masse est une technique classiquement utilisée pour détecter et pour identifier des espèces chimiques ou biologiques d'intérêt, telles que des molécules, des virus et des bactéries, évoluant dans un environnement liquide ou gazeux par mesure de leur masse. Dans le domaine médical, la spectrométrie de masse est un moyen efficace pour identifier et pour quantifier la présence de certains types d'espèces dans le corps humain, par exemple pour déterminer le taux de glucose dans le sang, pour identifier la présence de certaines substances illicites ou pour détecter des marqueurs tumoraux de certains cancers à des stades très précoces. Les dispositifs mettant en oeuvre une spectrométrie de masse sont généralement coûteux, volumineux et complexes à réaliser, ce qui rend leur déploiement et leur utilisation très restreints.

Au cours des dernières années, le recours à la photonique pour réaliser des dispositifs mettant en oeuvre une spectrométrie de masse a connu un intérêt croissant. Un tel intérêt s'explique par la maturité des technologies requises pour fabriquer des dispositifs manipulant la lumière qui répondent aux contraintes de coût et d'encombrement, et par le large champ d'applications qu'offre l'interaction lumière-matière dans le contexte de la spectrométrie de masse. Le principe de fonctionnement d'un dispositif photonique mettant en oeuvre une spectrométrie de masse repose généralement sur le changement de la réponse d'un composant optique suite à son interaction avec des espèces externes. Un tel changement est quantifiable en analysant les caractéristiques d'un signal optique interagissant avec un tel composant optique. A titre d'exemple, le micro-résonateur optique en anneau est un résonateur optique à fort potentiel en matière de détection d'espèces grâce notamment à ses conditions de résonance qui dépendent de la concentration d'espèces à proximité de sa surface active, la géométrie de la surface active étant une couronne circulaire entourant le micro-résonateur en anneau. Généralement, une couche de polymère actif est déposée sur la surface active d'un micro-résonateur en anneau de sorte à réagir chimiquement avec les espèces à sa proximité, entraînant ainsi un changement dans les conditions de résonance du micro-résonateur en anneau. Malgré son efficacité, un micro-résonateur en anneau présente l'inconvénient de fonctionner de manière indirecte en requérant une surface active à sa circonférence, et d'avoir une surface active qui se limite à la couronne circulaire qui l'entoure sans englober sa région intérieure, des espèces posées dans la région intérieure n'agissant presque pas sur les conditions de résonance du micro-résonateur en anneau et restant donc indétectables. Par exemple, lorsque l'espèce à détecter est présente en faible concentration dans l'environnement du micro-résonateur en anneau, l'étroitesse de la surface active rend la détection difficile à réaliser, un temps de mesure inversement proportionnel à la concentration de l'espèce d'intérêt étant requis.

En alternative à l'utilisation d'un micro-résonateur en anneau, il est connu d'utiliser un résonateur opto-mécanique comme composant optique de détection d'espèces. Agissant à la fois comme résonateur optique et résonateur mécanique en interaction réciproque et permanente, le résonateur opto-mécanique permet, en analysant le décalage de la longueur d'onde de résonance du résonateur optique, de remonter au mouvement du résonateur mécanique. Un tel couplage opto-mécanique est exploité par la communauté scientifique pour détecter toute espèce venant se poser sur la surface du résonateur opto-mécanique, ce qui a pour effet de changer la masse et/ou les contraintes de surface du résonateur opto-mécanique, et donc de changer sa fréquence de résonance mécanique. La détection d'espèces au moyen d'un résonateur opto-mécanique peut ainsi consister à suivre le décalage en fréquence de sa résonance mécanique au moyen d'un circuit à boucle fermée telle qu'une boucle à phase asservie (« Phase-loocked loop »). Cependant, les performances des résonateurs opto-mécaniques, en termes de sensibilité par exemple, sont fortement dépendantes de leurs facteurs de qualité mécanique et optique. Pour répondre à de telles contraintes, un résonateur opto-mécanique est généralement réalisé à partir d'une micro-cavité optique dont la longueur effective est modulée avec des vibrations mécaniques, la micro-cavité optique pouvant par exemple avoir la forme géométrique d'un anneau, d'un disque, d'un cristal photonique, etc. Les disques opto-mécaniques sont particulièrement intéressants comme ils présentent un fort facteur de qualité mécanique, typiquement compris entre 10 et 50, dans des environnements liquides. Le facteur de qualité des disques opto-mécaniques dans des environnements liquides est par exemple plus élevé que celui permis par des cantilevers qui mettent en oeuvre des modes de vibration en flexion et résonnent dans une gamme de fréquence 10 à 100 fois inférieure. Cependant, les dimensions micrométriques d'un disque opto-mécanique (quelques micromètres de rayon et environ 220 nanomètres d'épaisseur) sont généralement négligeables par rapport à celles de l'environnement dans lequel l'espèce à détecter évolue, et elles impliquent donc une surface active réduite. Ainsi, même pour une espèce d'intérêt présente en forte concentration dans l'environnement du disque opto-mécanique, un temps de mesure non négligeable, typiquement plusieurs dizaines de secondes, est requis pour détecter l'espèce d'intérêt. Le temps de mesure est d'autant plus long que la concentration de l'espèce à détecter est faible. Ceci constitue un obstacle majeur au déploiement d'un dispositif de détection à base d'un disque opto-mécanique, surtout dans des systèmes temps-réel requérant des mesures quasi-instantanées.

Le document US 2016/246000 décrit un dispositif de détection d'espèces chimiques ou biologique comprenant une matrice de résonateurs opto-mécaniques. Chacun de ces résonateurs présente une fréquence optique de résonance différente, leur adressage se faisant par multiplexage de longueur d'onde. La réalisation d'un tel dispositif est complexe, car les tolérances de fabrication rendent difficile un contrôle précis de la fréquence optique de résonance d'un résonateur micrométrique.

Il existe ainsi un besoin pour un dispositif et un procédé de détection d'espèces chimiques ou biologiques améliorés.

### Résumé de l'invention

Un objet de l'invention est un dispositif de détection configuré pour détecter des espèces chimiques ou biologiques dans un environnement donné, le dispositif de détection comprenant :
- un capteur matriciel comprenant :
- une pluralité de guides d'onde optiques, une pluralité d'électrodes de polarisation et une pluralité d'électrodes d'actionnement ;
- un ensemble de disques opto-mécaniques agencés en lignes et en colonnes, les disques d'une même ligne étant couplés optiquement avec un même guide d'onde optique, chaque disque opto-mécanique étant optiquement et mécaniquement résonant et étant apte à se lier à des espèces de l'environnement, les électrodes d'actionnement étant configurés pour assurer la résonance mécanique des disques opto-mécaniques ;
- une pluralité de jonctions PN, chaque jonction PN étant associée à un disque opto-mécanique, les jonctions PN d'une même colonne étant électriquement connectées à une même électrode de polarisation, chaque jonction PN étant configurée pour bloquer la circulation à travers le disque opto-mécanique correspondant d'un courant électrique parasite provenant d'une source autre que l'électrode de polarisation à laquelle la jonction PN est connectée ;
- une unité d'émission comprenant au moins une source laser configurée pour générer un signal optique porté à une longueur d'onde d'émission, l'unité d'émission étant en outre configurée pour injecter le signal optique dans les guides d'onde optiques du capteur matriciel ;
- un circuit de contrôle configuré pour polariser en direct, pendant une fenêtre temporelle de lecture d'un disque d'intérêt, la jonction PN du disque d'intérêt de manière à venir placer, par effet thermo-optique, sa longueur d'onde de résonance à une longueur d'onde de fonctionnement, ladite longueur d'onde de fonctionnement étant choisie de manière à ce que l'intensité du signal optique se propageant dans le guide d'onde optique associé au disque d'intérêt soit modulée par ledit disque d'intérêt, ce qui fournit un signal optique modulé ; et
- un circuit de lecture configuré pour déterminer à partir du signal optique modulé un résultat local de détection, pendant la fenêtre temporelle de lecture du disque d'intérêt.

Un autre objet de l'invention est un procédé de détection d'espèces chimiques ou biologiques utilisant un tel dispositf, le procédé de détection comprenant les étapes consistant à :
- injecter dans le guide d'onde optique du dispositif un signal optique porté à une longueur d'onde d'émission, le signal optique n'étant modulé par aucun disque opto-mécanique ;
- placer, pendant une fenêtre temporelle de lecture d'un disque d'intérêt du dispositif, la longueur d'onde de résonance du disque d'intérêt à une longueur d'onde de fonctionnement permettant la modulation du signal optique par le disque opto-mécanique d'intérêt, ce qui fournit un signal optique modulé ;
- déterminer un résultat local de détection à partir du signal optique modulé ;
- décaler, après l'écoulement de la fenêtre temporelle de lecture du disque d'intérêt, la longueur d'onde de résonance du disque opto-mécanique d'intérêt de manière à ce que le signal optique ne soit pas modulé par le disque opto-mécanique d'intérêt ;

les étapes b. à d. étant réitérées pour chacun des disques opto-mécaniques, ce qui fournit une pluralité de résultant locaux de détection.

Encore un autre objet de l'invention est un procédé de fabrication d'un capteur matriciel pour la réalisation d'un tel dispositif utilisant une plaque de matériaux semi-conducteurs comprenant un empilement d'une couche épaisse de silicium, d'une couche d'isolant et d'une couche fine de silicium, le procédé de fabrication comprenant les étapes suivantes :
- réaliser des clous métalliques de manière à assurer des connexions électriques entre les deux couches de silicium de la plaque ;
- réaliser des jonctions PN dans la couche fine de silicium, chaque jonction PN étant en contact avec un clou métallique ;
- former des coupleurs optiques dans la couche fine de silicium ;
- former des disques opto-mécaniques, leurs électrodes d'actionnement et des guides d'onde optiques ;
- réaliser des accès métalliques aux disques opto-mécaniques et aux électrodes d'actionnement ;
- libérer des éléments du capteur matriciel réalisés à partir de la couche fine de silicium.

### Brève description des figures

D'autres caractéristiques et avantages de la présente invention apparaîtront mieux à la lecture de la description qui suit en relation avec les dessins annexés suivants :
- La Figure 1 représente un dispositif de détection d'espèces chimiques ou biologiques, selon un mode de réalisation de l'invention ;
- La Figure 2 illustre le fonctionnement d'un disque opto-mécanique, selon des modes de réalisation de l'invention ;
- Les Figures 3-A à 3-E représentent l'évolution de la réponse optique des disques opto-mécaniques, selon un mode de réalisation de l'invention ;
- Les Figures 4-A à 4-D représentent l'évolution de la réponse optique des disques opto-mécaniques, selon un autre mode de réalisation de l'invention ;
- La Figure 5 représente un dispositif de détection d'espèces chimiques ou biologiques, selon un autre mode de réalisation de l'invention ;
- Les Figures 6-A et 6-B représentent une vue en coupe d'un disque opto-mécanique, selon deux modes de réalisation différents de l'invention ;
- La Figure 7 représente une vue en coupe d'un disque opto-mécanique, selon d'autres modes de réalisation de l'invention ;
- La Figure 8 est un organigramme représentant un procédé de détection d'espèces chimiques ou biologiques, selon des modes de réalisation de l'invention ;
- La Figure 9 est un organigramme représentant un procédé de fabrication d'un capteur matriciel, selon des modes de réalisation de l'invention ; et
- Les Figures 10-A à 10-E illustrent la sortie de certaines étapes du procédé de fabrication d'un capteur matriciel.

### Description détaillée

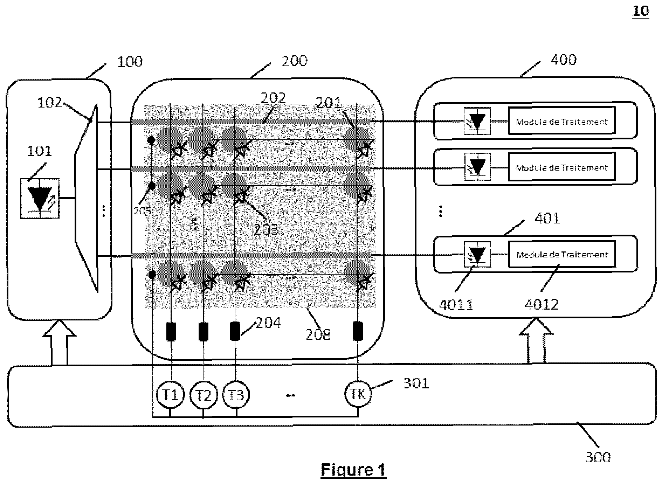

La figure 1 représente un dispositif de détection d'espèces chimiques ou biologiques 10 selon des modes de réalisation de l'invention. Le dispositif de détection 10 peut être déployé dans un environnement donné afin de détecter la présence d'un ou de plusieurs types d'espèces chimiques ou biologiques susceptibles d'évoluer dans l'environnement considéré. Le dispositif de détection 10 peut en outre être configuré pour déterminer d'autres caractéristiques en relation avec un type d'espèces détecté, telles que la concentration dans l'environnement considéré du type d'espèces détecté.

Le dispositif de détection 10 selon les modes de réalisation de l'invention peut être déployé dans un environnement liquide ou gazeux. Les particules compatibles avec le dispositif de détection 10 peuvent comprendre, par exemple et sans limitation, les atomes, les molécules, les ions, les protéines, les acides nucléiques, les virus, etc.

Dans un exemple d'application de l'invention dans le domaine médical, le dispositif de détection 10 peut être utilisé pour mettre en oeuvre des analyses sanguines afin de déterminer la concentration dans le sang d'un type donné d'espèces, par exemple pour déterminer le taux de glucose (glycémie) dans le sang, pour déterminer le groupe sanguin d'un patient ou pour détecter la présence d'anticorps dirigés contre un type donné de virus tel que le SARS-CoV-2.

Dans un autre exemple d'application de l'invention, le dispositif de détection 10 peut être mis en oeuvre dans des systèmes de surveillance destinés à déclencher des alarmes en fonction de la concentration d'un type d'espèces chimiques ou biologiques dans un environnement donné, tels qu'un système de détection du monoxyde de carbone dans des environnements fermés et un système d'évaluation de la qualité de l'air ambiant.

Comme représenté sur la figure 1, le dispositif de détection 10 comprend une unité d'émission 100, l'unité d'émission 100 comprenant une source laser 101 configurée pour générer un signal optique à une longueur d'onde d'émission donnée. La source laser 101 peut ne pas être modulée. Dans ce cas, des caractéristiques du signal optique généré tels que son intensité, sa longueur d'onde et son état de polarisation restent constantes au cours du temps.

Dans un mode de réalisation de l'invention, la source laser 101 utilisée dans l'unité d'émission 100 peut être une source laser à longueur d'onde constante au cours du temps.

Dans un autre mode de réalisation de l'invention, la source laser 101 utilisée dans l'unité d'émission 100 peut être une source laser accordable en longueur d'onde. Dans ce cas, la source laser 101 peut être configurée pour recevoir un signal de commande permettant de déterminer la longueur d'onde d'émission à laquelle le signal optique est généré.

Le dispositif de détection 10 comprend en outre un capteur matriciel 200, le capteur matriciel 200 étant configuré pour recevoir le signal optique généré par l'unité d'émission 100 via une connexion filaire ou via l'espace libre. Dans le mode de réalisation où la connexion entre l'unité d'émission 100 et le capteur matriciel 200 est de type filaire, un guide d'onde optique 202, tel qu'une fibre optique ou un guide d'onde intégré au substrat, peut être utilisé pour assurer l'acheminement du signal optique de l'unité d'émission 100 vers le capteur matriciel 200.

Le capteur matriciel 200 peut comprendre un agencement bidirectionnel de disques opto-mécaniques 201, les disques opto-mécaniques 201 étant arrangés en lignes et en colonnes. L'agencement bidirectionnel de disques 201 peut être géométriquement régulier, c'est-à-dire que les lignes du capteur matriciel 200 comprennent un même nombre de disques opto-mécaniques 201 et que la distance séparant deux disques 201 voisins d'une même ligne est égale à la distance séparant deux disques 201 voisins d'une même colonne. Les disques opto-mécaniques 201 peuvent en outre être suspendus parallèlement à un substrat commun 208 au moyen de supports dédiés 207.

Un disque opto-mécanique 201 sera désigné ci-après par Dᵢⱼ, les indices i et j désignant respectivement la i^{éme} ligne et la j^{éme} colonne du capteur matriciel 200 auxquelles le disque opto-mécanique 201 appartient.

Chacun des disques opto-mécaniques 201 Dᵢⱼ est optiquement résonant, c'est-à-dire qu'un signal optique interagissant avec le disque opto-mécanique 201 peut subir, lorsqu'il est caractérisé en transmission, une atténuation en fonction de sa longueur d'onde, l'atténuation provenant des interférences avec le signal optique incident après un tour dans le disque opto-mécanique, de l'absorption et de la diffusion de lumière par le disque opto-mécanique. L'atténuation est maximale lorsque la longueur d'onde du signal optique coïncide avec une longueur d'onde de résonance, notée λ_{rés}, caractérisant le disque opto-mécanique 201. L'atténuation est minimale pour certaines plages de longueurs d'onde du signal optique, ce qui correspond à un signal optique placé hors de la résonance du disque opto-mécanique 201. La longueur d'onde de résonance λ_{rés} d'un disque 201 Dᵢⱼ dépend de plusieurs paramètres opto-géométriques du disque opto-mécanique 201, tels que par exemple son rayon et son indice de réfraction effectif.

Chacun des disques opto-mécaniques 201 Dᵢⱼ est mécaniquement résonant, c'est-à-dire que chaque disque 201 Dᵢⱼ est soumis à une modulation externe faisant varier son rayon, le disque 201 étant donc soumis à une succession d'expansions et de contractions. La modulation externe peut s'effectuer à une amplitude constante au cours du temps et à une fréquence de résonance mécanique, noté Fₘ, pouvant atteindre quelques GHz. Ainsi, la modulation externe d'un disque opto-mécanique 201 Dᵢⱼ fait également moduler sa longueur d'onde de résonance λ_{rés} qui dépend de son rayon.

Avantageusement, les disques opto-mécaniques 201 Dᵢⱼ mis en oeuvre dans le capteur matriciel 200 peuvent être fabriqués à partir d'un matériau ou à partir d'un alliage de plusieurs matériaux en tenant compte de la longueur d'onde d'émission de la source laser 101 et de l'indice de réfraction de l'environnement dans lequel le dispositif de détection 10 est destiné à être déployé. De manière générale, les matériaux formant les disques opto-mécaniques 201 doivent être transparents à la longueur d'onde d'émission de la source laser 101 tout en permettant un contraste d'indice élevé par rapport à l'environnement. En outre, les caractéristiques géométriques des disques opto-mécaniques 201 telles que leur rayon et leur épaisseur peuvent être choisies de manière à permettre l'existence d'un ou de plusieurs modes optiques confinés. Dans un exemple où le dispositif de détection 10 fonctionne dans l'infrarouge et est destiné à être déployé dans un environnement gazeux, tel que l'air ambiant, les disques opto-mécaniques 201 peuvent être fabriqués à base de silicium (indice de réfraction égal à 3,4). Dans un autre exemple où le dispositif de détection 10 fonctionne dans l'infrarouge et est destiné à être déployé dans un environnement liquide, tel que l'eau, les disques opto-mécaniques 201 peuvent être fabriqués à base de silicium ou à base d'Arséniure de Gallium (indice de réfraction supérieur à 4), à titre d'exemple non limitatif.

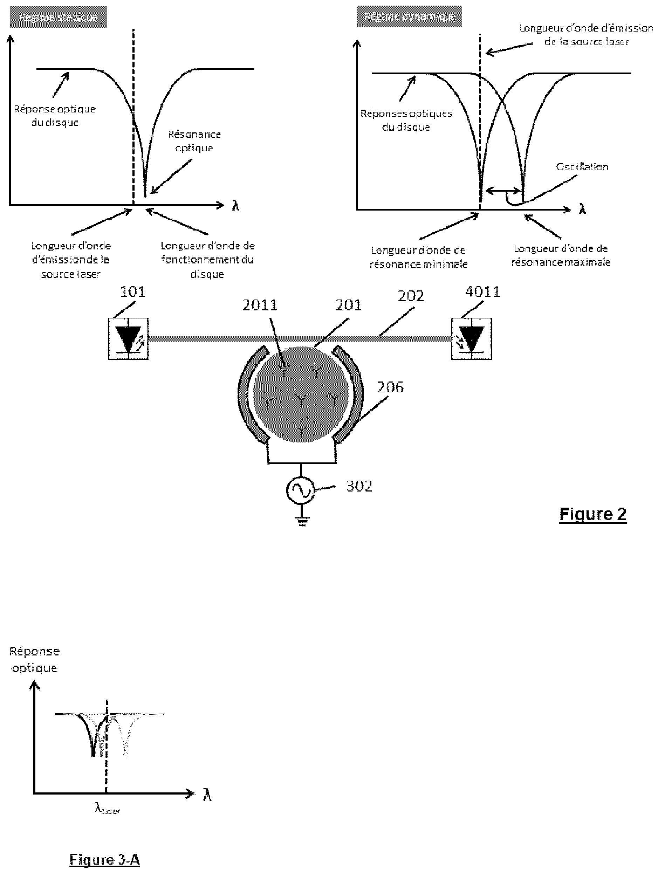

Le capteur matriciel 200 comprend en outre une pluralité de récepteurs 2011 (représentés dans la figure 2) disposés de manière sensiblement uniforme sur la face de détection de chacun des disques opto-mécaniques 201, la face de détection d'un disque opto-mécanique 201 étant définie comme étant la face qui n'est pas en contact avec le support 207 reliant le disque 201 au substrat commun 208. Les récepteurs 2011 sont alors configurés de manière à se lier uniquement à des espèces chimiques ou biologiques d'intérêt à détecter par le dispositif de détection 10. La liaison entre un récepteur 2011 et une espèce d'intérêt peut s'effectuer au moyen des forces s'exerçant entre molécules, telles que des liaisons ioniques, des liaisons d'hydrogène et des forces de van der Waals. Lorsque des espèces sont liées à des récepteurs 2011 d'un disque opto-mécanique 201, la masse et/ou les contraintes de surface changent et induisent un changement de la fréquence de résonance mécanique du disque opto-mécanique 201. Par ailleurs, la liaison entre un récepteur 2011 et une espèce d'intérêt peut être réversible, ce qui signifie qu'il est possible de libérer les récepteurs 2011 de leurs particules au moyen d'un procédé dédié sans détériorer l'état des récepteurs 2011 mis en oeuvre. Dans le cas où le dispositif de détection 10 est destiné à détecter un seul type d'espèces, les récepteurs 2011 mis en oeuvre dans le capteur matriciel 200 peuvent être identiques.

Alternativement, le dispositif de détection 10 peut être configuré pour détecter deux types différents d'espèces. Dans un tel mode de réalisation, le capteur matriciel 200 peut comprendre deux types différents de récepteurs, chaque type de récepteurs 2011 étant configuré pour se lier à un type différent d'espèces. Avantageusement, les capteurs d'un même type peuvent être disposés sur les disques opto-mécaniques 201 d'une même ligne, pour faciliter la lecture des disques opto-mécaniques 201. L'homme du métier comprendra aisément que le dispositif de détection d'espèces chimiques ou biologiques 10 peut être configuré pour détecter autant de types différents d'espèces que de lignes de disques opto-mécaniques 201.

Le capteur matriciel 200 comprend en outre une pluralité de guides d'onde optiques 202, chaque guide d'onde optique 202 étant associé à une ligne de disques opto-mécaniques 201 en étant optiquement couplé avec tous les disques 201 de la ligne associée. Le couplage optique entre un guide d'onde optique 202 et les disques 201 de la ligne associée peut s'effectuer selon un même coefficient de couplage optique. Le coefficient de couplage optique entre un guide d'onde optique 202 et un disque opto-mécanique 201 est fonction de la distance minimale qui sépare le guide d'onde et le disque, ce qui rend l'ajustement d'un coefficient de couplage difficilement réalisable après la fabrication du capteur matriciel 200.

L'unité d'émission 100 peut en outre comprendre un module optique de répartition 102 configuré pour injecter le signal optique généré par la source laser 101 dans un ou dans plusieurs guides d'onde optiques 202.

Dans des modes de réalisation de l'invention, le module optique de répartition 102 peut être configuré pour injecter, simultanément, dans chaque guide d'onde optique 202 du capteur matriciel 200 une portion du signal optique généré par la source laser 101. Par exemple, le module optique de répartition 102 peut être de type coupleur optique à une entrée et à N sorties, N étant le nombre de lignes du capteur matriciel 200. Un coupleur optique présente l'avantage d'être passif et de ne pas requérir une alimentation électrique.

Dans d'autres modes de réalisation de l'invention, le module optique de répartition 102 peut être configuré pour injecter, à un instant donné, la totalité du signal optique généré par la source laser 101 dans une ligne d'intérêt du capteur matriciel 200. Dans ce cas, le module optique peut en outre être configuré pour recevoir un signal de commande afin de déterminer la ligne d'intérêt parmi les N lignes possibles. Par exemple, le module optique de répartition 102 peut être de type commutateur optique à une entrée et à N sorties. De tels modes de réalisation sont particulièrement avantageux dans le cas où le nombre de lignes du capteur matriciel 200 est élevé, typiquement supérieur à 20.

Le capteur matriciel 200 comprend une pluralité d'électrodes de polarisation 204, chaque électrode de polarisation 204 étant associée à une colonne du capteur matriciel 200. En outre, les disques opto-mécaniques 201 appartenant à une même colonne peuvent être électriquement connectés à l'électrode de polarisation 204 associée au moyen d'un conducteur métallique 209 qui peut être à base de cuivre, par exemple. Le capteur matriciel 200 comprend en outre une électrode commune 205 à tous les disques opto-mécaniques 201 mis en oeuvre dans le capteur matriciel 200. Avantageusement, l'électrode commune 205 peut être agencée sur le capteur matriciel 200 de manière à permettre la circulation, à travers les disques opto-mécaniques 201 d'une colonne quelconque du capteur matriciel 200, d'un courant électrique issu d'une différence de potentiel électrique entre l'électrode de polarisation 204 associée à la colonne et l'électrode commune 205. Lorsqu'un courant électrique traverse un disque opto-mécanique 201, le disque 201 s'échauffe et son indice de réfraction varie. Ceci a pour conséquence le décalage de la longueur d'onde de résonance du disque opto-mécanique 201.

Le capteur matriciel 200 comprend en outre une pluralité de jonctions PN 203, chaque jonction PN 203 étant associée à un disque opto-mécanique 201 et comprenant une région dopée P et une région dopée N. En outre, chaque jonction PN 203 d'un disque opto-mécanique 201 peut être agencée dans le capteur matriciel 200 de manière à ce que sa région dopée P soit électriquement connectée à l'électrode de polarisation 204 associée au disque opto-mécanique 201 et à ce que sa région dopée N soit électriquement connectée à l'électrode commune 205. Une telle configuration présente l'avantage de permettre la circulation à travers le disque opto-mécanique 201 de tout courant électrique provenant de l'électrode de polarisation 204 associé au disque 201 (polarisation en direct de la jonction PN 203), et de bloquer la circulation à travers le disque opto-mécanique 201 de tout courant électrique parasite provenant d'autres sources, en particulier les courants électriques parasites provenant de l'électrode commune 205 (polarisation en inverse de la jonction PN 203). Ainsi, en considérant les disques opto-mécaniques 201 d'une ligne d'intérêt du capteur matriciel 200, il est possible, en polarisant en direct la jonction PN 203 d'un disque opto-mécanique d'intérêt 201, de décaler sa longueur d'onde de résonance sans que les autres disques 201 de la ligne d'intérêt ne soient traversés par des courants électriques parasites. En polarisant en direct et de manière indépendante la jonction PN 203 de chacun des disques opto-mécaniques 201, il est possible de décaler séparément les longueurs d'onde de résonance des disques opto-mécaniques 201 de chaque ligne du capteur matriciel 200. Ainsi, il est possible de configurer les disques 201 d'une ligne d'intérêt pour qu'un seul disque 201 de la ligne interagisse avec le signal optique injecté dans le guide d'onde optique 202, le signal optique étant hors résonance pour les autres disques 201 de la ligne. Ceci permet d'avoir à la sortie du guide d'onde optique 202 un signal optique exploitable, c'est-à-dire détectable par un photo-détecteur 4011. Une telle caractéristique du dispositif de détection 10 est particulièrement avantageuse lorsque le nombre de disques opto-mécaniques 201 agencés sur chaque ligne est élevé, typiquement supérieur à 50.

Les solutions de l'état de la technique en matière de contrôle, par effet thermo-optique, des conditions de résonance d'un résonateur optique ou opto-mécanique mettent généralement en oeuvre une résistance chauffante à proximité du résonateur à contrôler. Malgré une efficacité approuvée dans une configuration comprenant un seul résonateur tel que décrit dans [1], une telle solution est incompatible avec un réseau de résonateurs où un nombre significatif, supérieur à 100 par exemple, de résonateurs sont agencés sur une surface d'intégration de quelques centimètres carrés. En effet, la nature symétrique de la résistance chauffante qui laisse passer des courants électriques dans les deux sens favorise l'apparition de courants électriques parasites dans le réseau de résonateurs. De tels courants électriques parasites perturbent le fonctionnement du réseau de résonateurs en modifiant arbitrairement les conditions de résonance de certains résonateurs optiques mis en oeuvre. Par ailleurs, la résistance chauffante est généralement séparée du résonateur à contrôler par un empilement de plusieurs couches de semi-conducteurs et/ou de métaux, ceci implique une puissance électrique élevée pour contrôler les conditions de résonance du résonateur en utilisant les solutions de l'état de la technique.

La Figure 2 illustre le fonctionnement d'un disque opto-mécanique 201, selon des modes de réalisation de l'invention. La réponse optique du disque opto-mécanique 201 se présente sous une forme lorentzienne associée à une atténuation maximale à la longueur d'onde de résonance et à deux fronts : un front montant et un front descendant. La longueur d'onde de résonance du disque opto-mécanique 201 peut être préalablement placée, par exemple pendant un régime statique sans modulation externe, à une longueur d'onde de fonctionnement, la longueur d'onde de fonctionnement étant choisie de manière à ce que la longueur d'onde du signal optique d'entrée reste sur un même front, montant ou descendant, pendant l'oscillation de la réponse optique du disque 201, c'est-à-dire pendant un régime dynamique avec modulation externe. Dans un régime dynamique consistant à soumettre le disque opto-mécanique 201 a une modulation externe à la fréquence de résonance mécanique Fₘ afin de le rendre vibrant, la réponse optique du disque opto-mécanique 201 oscille selon le même rythme, c'est-à-dire à la fréquence de résonance mécanique Fₘ. En particulier, la longueur d'onde de résonance du disque opto-mécanique 201 oscille entre une longueur d'onde de résonance minimale et une longueur d'onde de résonance maximale. Dans ce cas, l'atténuation subie par le signal optique d'entrée (équivalente à la perte introduite par le disque) évolue quasi-linéairement à chaque demi-période d'oscillation de la réponse optique du disque, le signal optique de sortie étant ainsi modulé en intensité. Optionnellement, la longueur d'onde de fonctionnement peut en outre être optimisée de manière à obtenir une puissance optique maximale du signal optique de sortie. Dans un mode de réalisation, la modulation externe appliquée au disque opto-mécanique 201 peut être réalisée au moyen de deux électrodes d'actionnement 206 entourant le disque opto-mécanique 201 et connectées à un générateur de signaux radiofréquences 302. Dans ce cas, les deux électrodes d'actionnement 206 génèrent une force d'actionnement électrostatique permettant d'entraîner une vibration du disque opto-mécanique 201.

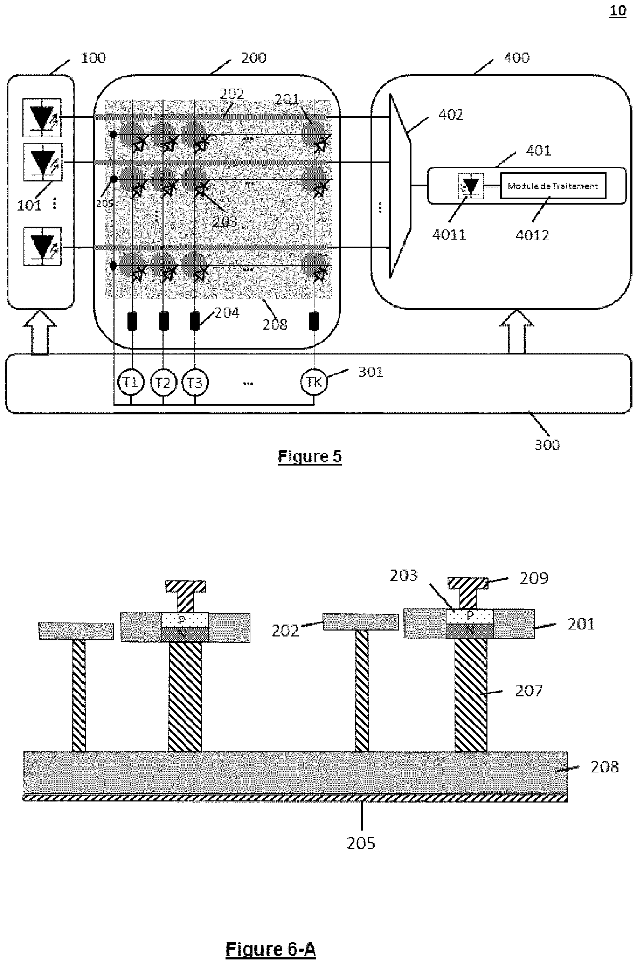

Avantageusement, le capteur matriciel 200 peut en outre comprendre plusieurs électrodes d'actionnement 206 (non représentées sur les figures 1 et 5), chacun des disques opto-mécaniques 201 du capteur matriciel 200 étant entouré par au moins deux électrodes d'actionnement 206 tel que décrit en relation avec la figure 2. Le circuit de contrôle 300 comprend en outre un générateur de signaux radiofréquences 302 configuré pour fournir aux électrodes d'actionnement 206 entourant chaque disque 201 un signal de modulation permettant de soumettre chaque disque opto-mécanique 201 à une force d'actionnement électrostatique entraînant sa vibration.

Le dispositif de détection 10 comprend en outre un circuit de contrôle 300 configuré pour contrôler le fonctionnement d'un ou de plusieurs éléments constitutifs du dispositif de détection 10. De tels éléments constitutifs peuvent comprendre le capteur matriciel 200 et l'unité d'émission 100. Le circuit de contrôle 300 comprend des sources de tension électrique réglables 301 qui peuvent correspondre en nombre au nombre de colonnes dans le capteur matriciel 200, chaque source de tension électrique réglable 301 pouvant être connectée à l'électrode de polarisation 204 d'une colonne correspondante dans le capteur matriciel 200 et à l'électrode commune 205. Dans le cas où le module optique de répartition 102 mis en place dans l'unité d'émission 100 est de type commutateur optique, le circuit de contrôle 300 peut être configuré pour transmettre au module optique de répartition 102, préalablement aux fenêtres temporelles de lecture des disques 201 d'une ligne d'intérêt, un signal de commande permettant d'injecter un signal optique dans le guide d'onde optique 202 de la ligne d'intérêt.

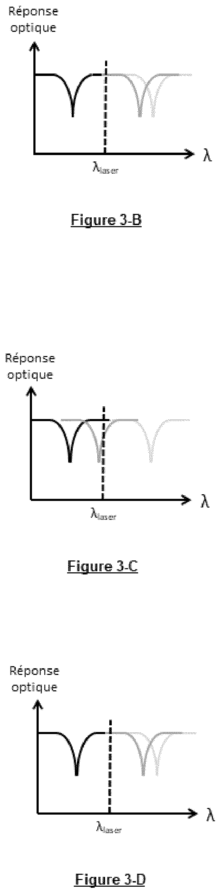

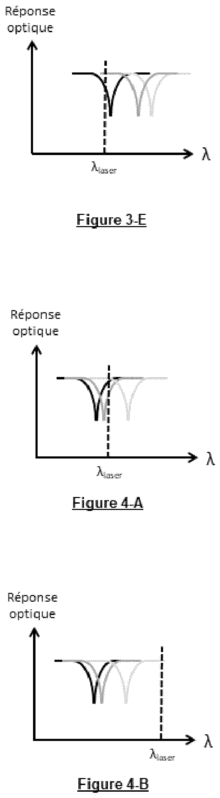

Dans des modes de réalisation de l'invention où la source laser 101 mise en oeuvre dans l'unité d'émission 100 n'est pas accordable en longueur d'onde, le circuit de contrôle 300 peut être configuré pour mettre en oeuvre, préalablement aux fenêtres temporelles de lecture des disques 201 d'une ligne d'intérêt, une première étape consistant à décaler la longueur d'onde de résonance d'un ou de plusieurs disques 201 appartenant à la ligne d'intérêt de manière à placer le signal optique qui se propage dans le guide d'onde optique 202 hors de la résonance de chacun des disques de la ligne d'intérêt, c'est-à-dire qu'aucun disque 201 de la ligne d'intérêt ne module le signal optique qui se propage dans le guide d'onde optique 202. Le circuit de contrôle 300 peut être configuré pour ensuite mettre en oeuvre une deuxième étape consistant à placer, pendant une fenêtre temporelle de lecture d'un disque d'intérêt 201 appartenant à la ligne d'intérêt, la longueur d'onde de résonance du disque d'intérêt 201 à une longueur d'onde de fonctionnement permettant la modulation de l'intensité du signal optique tel que décrit en relation avec la figure 2. Le disque d'intérêt 201 est soumis à une modulation externe, ce qui fournit un signal optique modulé en intensité à la sortie du guide d'onde optique 202. Après l'écoulement de la fenêtre temporelle de lecture du disque d'intérêt 201, le circuit de contrôle 300 peut être configuré pour mettre en oeuvre de nouveau la première étape pour placer le signal optique qui se propage dans le guide d'onde optique 202 hors de la résonance de chacun des disques de la ligne d'intérêt. Avantageusement, le circuit de contrôle 300 peut être configuré pour mettre en oeuvre plusieurs itérations, chaque itération comprenant une exécution de la première et de la deuxième étape, pour permettre la lecture d'autres disques 201 de la ligne d'intérêt, la lecture s'effectuant de manière individuelle et séquentielle dans le temps. Les figures 3-A à 3-E illustrent, à titre d'exemple, l'ajustement des longueurs d'onde de résonance des disques opto-mécaniques 201 d'une ligne d'intérêt afin de permettre la lecture de tous les disque 201 de la ligne d'intérêt, selon de tels modes de réalisation. Plus précisément, la figure 3-A correspond à un état initial des disques opto-mécaniques 201 selon lequel les longueurs d'onde de résonance des disques 201 sont arbitrairement dispersées sur un large spectre de longueurs d'onde, la longueur d'onde d'émission de la source laser 101 étant, par exemple et sans limitation, soumise à l'atténuation d'un ou de plusieurs disques opto-mécaniques 201. La figure 3-B illustre la configuration des longueurs d'onde de résonance des disques 201 après la mise en oeuvre par le circuit de contrôle de la première étape décrite ci-dessus consistant à placer hors résonance le signal optique généré par la source laser en décalant la longueur d'onde de résonance d'un ou de plusieurs disques opto-mécaniques 201 par polarisation de la ou des jonctions PN correspondantes. La figure 3-C correspond à la configuration des longueurs d'onde de résonance des disques 201 après la mise en oeuvre de la deuxième étape décrite ci-dessus consistant à placer, pendant une fenêtre temporelle de lecture d'un disque d'intérêt, la longueur d'onde de résonance du disque d'intérêt à une longueur d'onde de fonctionnement tel que décrit en relation avec la figure 2. Les figures 3-D et 3-E correspondent à une nouvelle itération de la première et de la deuxième étape mises en oeuvre par le circuit de contrôle 300 afin de permettre la lecture d'un autre disque opto-mécaniques 201 appartenant à la ligne d'intérêt. De tels modes de réalisation présentent l'avantage d'utiliser une source laser 101 qui n'est pas accordable en longueur d'onde.

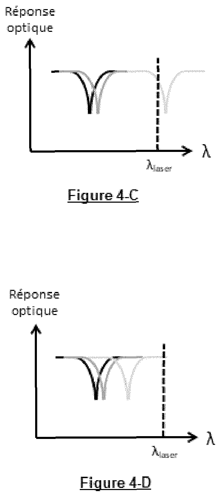

Dans d'autres modes de réalisation de l'invention où la source laser 101 mise en oeuvre dans l'unité d'émission 100 est accordable en longueur d'onde, le circuit de contrôle 300 peut être configuré pour mettre en oeuvre, préalablement aux fenêtres temporelles de lecture des disques 201 d'une ligne d'intérêt, une première étape consistant à déterminer la longueur d'onde d'émission de la source laser 101 de manière à placer le signal optique qui se propage dans le guide d'onde optique 202 hors de la résonance de chacun des disques de la ligne d'intérêt, c'est-à-dire qu'aucun disque 201 de la ligne d'intérêt ne module le signal optique qui se propage dans le guide d'onde optique 202. Le circuit de contrôle 300 peut être configuré pour mettre en oeuvre ensuite la deuxième étape décrite ci-dessus consistant à placer, pendant une fenêtre temporelle de lecture d'un disque d'intérêt 201 appartenant à la ligne d'intérêt, la longueur d'onde de résonance du disque d'intérêt 201 à une longueur d'onde de fonctionnement permettant la modulation de l'intensité du signal optique tel que décrit en relation avec la figure 2. Après l'écoulement de la fenêtre temporelle de lecture du disque d'intérêt 201, le circuit de contrôle 300 peut être configuré pour mettre en oeuvre une troisième étape consistant à placer le signal optique qui se propage dans le guide d'onde optique 202 hors de la résonance de chacun des disques de la ligne d'intérêt. Avantageusement, le circuit de contrôle 300 peut être configuré pour mettre en oeuvre plusieurs itérations, chaque itération comprenant une exécution de la deuxième et de la troisième étape, pour permettre la lecture d'autres disques 201 de la ligne d'intérêt, la lecture s'effectuant de manière individuelle et séquentielle dans le temps. Les figures 4-A à 4-D illustrent, à titre d'exemple, l'ajustement des longueurs d'onde de résonance des disques opto-mécaniques 201 d'une ligne d'intérêt afin de permettre la lecture de tous les disque 201 de la ligne d'intérêt, selon de tels modes de réalisation. Plus précisément, la figure 4-A correspond à un état initial des disques opto-mécaniques 201. La figure 4-8 illustre le décalage de la longueur d'onde d'émission de la source laser 101 selon la première étape décrite ci-dessus. La figure 4-C correspond à la configuration des longueurs d'onde de résonance des disques 201 après la mise en oeuvre de la deuxième étape décrite ci-dessus consistant à placer, pendant une fenêtre temporelle de lecture d'un disque d'intérêt, la longueur d'onde de résonance du disque d'intérêt à une longueur d'onde de fonctionnement tel que décrit en relation avec la figure 2. La figure 4-D correspond à la configuration des longueurs d'onde de résonance des disques 201 après la mise en oeuvre de la troisième étape décrite ci-dessus. De tels modes de réalisation présentent l'avantage de requérir la polarisation d'une seule jonction PN 203 à chaque fenêtre temporelle de lecture d'un disque d'intérêt 201.

Comme illustré sur la figure 1, le dispositif de détection 10 comprend en outre un circuit de lecture 400 configuré pour recevoir chaque signal optique modulé après propagation à travers le guide d'onde optique 202 associé. Le circuit de lecture 400 peut comprendre une pluralité d'unités de lecture 401, chacune des unités de lecture 401 étant associée à un guide d'onde optique 202 et comprenant un photo-détecteur 4011 et un module de traitement 4012. Le photo-détecteur 4011 est configuré pour convertir le signal optique modulé reçu dans le domaine électrique, ce qui fournit un signal électrique de détection. Le module de traitement 4012 est configuré pour déterminer un résultat local de détection à partir des caractéristiques du signal électrique de détection.

Avantageusement, le fonctionnement d'un module de traitement 4012 peut être régi par le circuit de contrôle 300 mis en oeuvre dans le dispositif de détection 10. Dans un tel mode de réalisation, le circuit de contrôle 300 peut être configuré, pendant une fenêtre temporelle de lecture d'un disque d'intérêt 201, pour placer la longueur d'onde de résonance du disque d'intérêt 201 à une longueur d'onde de fonctionnement tel que décrit en relation avec la figure 2, et pour déclencher la détermination d'un résultat local de détection par le module de traitement 4012 associé à la même ligne que le disque d'intérêt 201. Par exemple, le module de traitement 4012 peut être configuré, en réponse à la réception d'un signal électrique de détection, pour mettre en oeuvre les étapes consistant à :
- déterminer la fréquence de modulation Fₛₒᵣₜᵢₑ du signal électrique de détection ;
- calculer une différence de fréquences ΔF entre la fréquence de modulation Fₛₒᵣₜᵢₑ et une fréquence de référence F_{réf}.
- déterminer le résultat local de détection à partir de la différence de fréquences ΔF.

La fréquence de référence F_{réf} représente la fréquence de résonance mécanique du disque opto-mécanique d'intérêt 201 lorsqu'aucune espèce n'est liée à ses récepteurs 2011. La fréquence de modulation Fₛₒᵣₜᵢₑ représente la fréquence de résonance mécanique du disque opto-mécanique d'intérêt 201 lorsque des espèces sont susceptibles d'être liées à ses récepteurs 2011. De manière générale, la différence de fréquence ΔF est proportionnelle à la masse des espèces liées aux récepteurs 2011 du disque d'intérêt 201.

Dans des modes de réalisation de l'invention, le dispositif de détection d'espèces chimiques ou biologiques 10 peut être configuré pour mettre en oeuvre une lecture séquentielle des lignes du capteur matriciel 200, ce qui fournit pour chaque ligne du capteur matriciel 200 une pluralité de résultats locaux de détection. Le dispositif de détection 10 peut en outre être configuré pour déterminer un résultat global de détection à partir des pluralités de résultats locaux de détection ainsi déterminées. Pour chaque ligne du capteur matriciel 200, le dispositif de détection 10 peut être configuré pour réaliser une lecture séquentielle des disques opto-mécaniques 201 en mettant en oeuvre les étapes consistant à :
a. injecter dans le guide d'onde optique 202 de la ligne un signal optique porté à une longueur d'onde d'émission, le signal optique n'étant modulé par aucun disque opto-mécanique 201 de la ligne ;
b. placer, pendant une fenêtre temporelle de lecture d'un disque d'intérêt 201, la longueur d'onde de résonance du disque d'intérêt 201 à une longueur d'onde de fonctionnement permettant la modulation du signal optique par le disque opto-mécanique d'intérêt 201, ce qui fournit un signal optique modulé ;
c. déterminer un résultat local de détection à partir du signal optique modulé ;
d. décaler, après l'écoulement de la fenêtre temporelle de lecture du disque d'intérêt 201, la longueur d'onde de résonance du disque opto-mécanique d'intérêt 201 de manière à ce que le signal optique ne soit pas modulé par le disque opto-mécanique d'intérêt 201 ;

Le dispositif de détection 10 peut être configuré pour mettre en oeuvre les étapes b. à d. pour chacun des disques opto-mécaniques 201 de la ligne, ce qui fournit la pluralité de résultats locaux de détection qui sont obtenus de manière rapide dans le temps.

Dans un mode de réalisation, le dispositif de détection 10 peut être configuré pour mettre en oeuvre une phase préalable de calibrage consistant à déterminer la fréquence de référence F_{réf} de chacun des disques opto-mécaniques 201 mis en oeuvre dans le capteur matriciel 200, la fréquence de référence F_{réf} d'un disque opto-mécanique 201 étant sa fréquence de résonance mécanique lorsqu'aucune particule n'est liées à ses récepteurs 2011.

La figure 5 représente un dispositif de détection d'espèces chimiques ou biologiques 10, selon un autre mode de réalisation de l'invention. Dans un tel mode de réalisation, l'unité d'émission 100 peut comprendre une pluralité de sources laser 101. Par exemple, le nombre de sources laser 101 peut être égal au nombre de lignes du capteur matriciel 200. Dans ce cas, chaque source laser 101 est optiquement couplée avec un seul guide d'onde optique 202. Les sources laser 101 peuvent être à longueur d'onde accordable, la longueur d'onde d'émission de chacune des sources laser 101 étant déterminée par le circuit de contrôle 300 mis en oeuvre en fournissant des signaux de commande. L'utilisation d'une pluralité de sources laser 101 dans l'unité d'émission 100 permet de s'affranchir du module optique de répartition 102.

Comme illustré sur la figure 5, le circuit de lecture 400 peut comprendre une seule unité de lecture 401. Dans un tel mode de réalisation, le dispositif de détection 10 comprend en outre un commutateur optique 402 à N entrées et à une sortie commandé par le circuit de contrôle 300, chaque entrée optique du commutateur étant reliée à la sortie d'un guide d'onde optique 202 correspondant et la sortie du commutateur optique étant reliée à l'entrée optique du photo-détecteur 4011 mis en oeuvre dans l'unité de lecture 401. Le circuit de contrôle 300 peut être configuré pour relier, pendant une fenêtre temporelle de lecture d'un disque d'intérêt 201, la sortie du guide d'onde optique 202 associé au disque d'intérêt 201 avec l'unité de lecture 401, au moyen du commutateur optique.

La figure 6-A représente un disque opto-mécanique 201 et la jonction PN 203 correspondante, selon un mode de réalisation de l'invention. La jonction PN 203 est réalisée par dopage d'une région centrale du disque opto-mécanique 201, la région centrale dopée présentant une forme cylindrique et son rayon étant inférieur à celui du disque opto-mécanique 201. Par exemple, le rayon de la région centrale dopée peut être compris entre 50% et 90% du rayon du disque opto-mécanique 201. La région dopée P de la jonction PN 203 peut être réalisée de manière à ce qu'elle s'ouvre sur la face de détection du disque opto-mécanique 201. La région dopée N de la jonction PN 203 peut être réalisée de manière à couvrir toute la surface de contact entre le disque 201 et le support de disque 207.

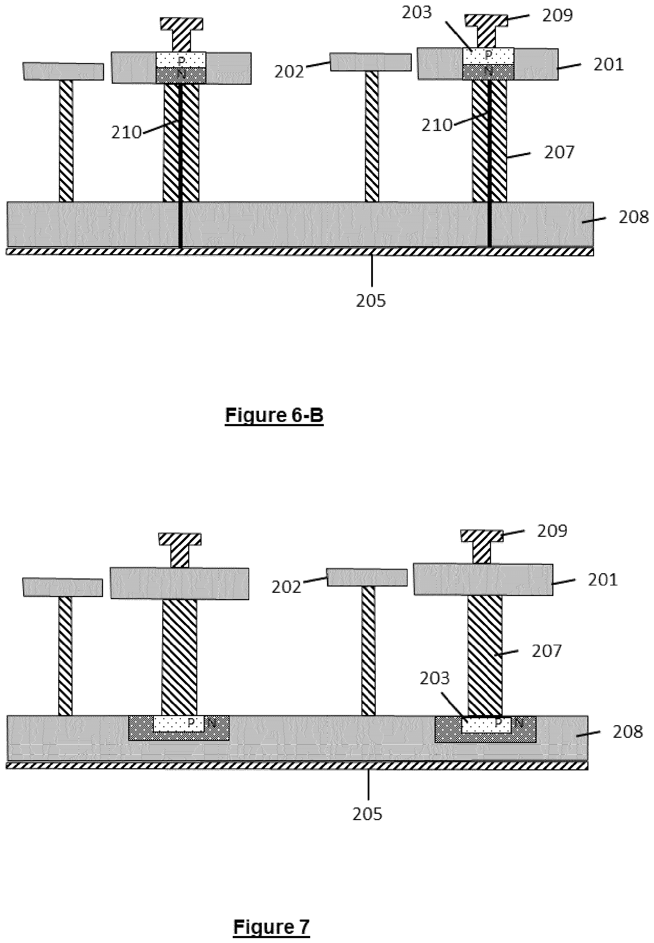

La figure 6-B représente un disque opto-mécanique 201 et la jonction PN 203 correspondante, selon un autre mode de réalisation de l'invention. En comparaison avec la structure décrite en relation avec la figure 6-A, la structure de la figure 6-B comprend en outre des clous métalliques 210. Chaque clou métallique 210 est associé à un disque opto-mécanique 201 et assure une connexion électrique entre le disque 201 correspondant et l'électrode commune 205.

La figure 7 représente un disque opto-mécanique 201 et la jonction PN 203 correspondante, selon d'autres modes de réalisation de l'invention. Dans de tels modes de réalisation, la jonction PN 203 associée à un disque opto-mécanique 201 est réalisée par dopage du substrat commun 208 sur lequel est posé le support 207 permettant de suspendre le disque opto-mécanique 201. La région dopée P de la jonction PN 203 peut être réalisée de manière à couvrir toute la surface de contact entre le support 207 du disque opto-mécanique 201 et le substrat commun 208. La région dopée N peut être réalisé de manière à contenir la région dopée P.

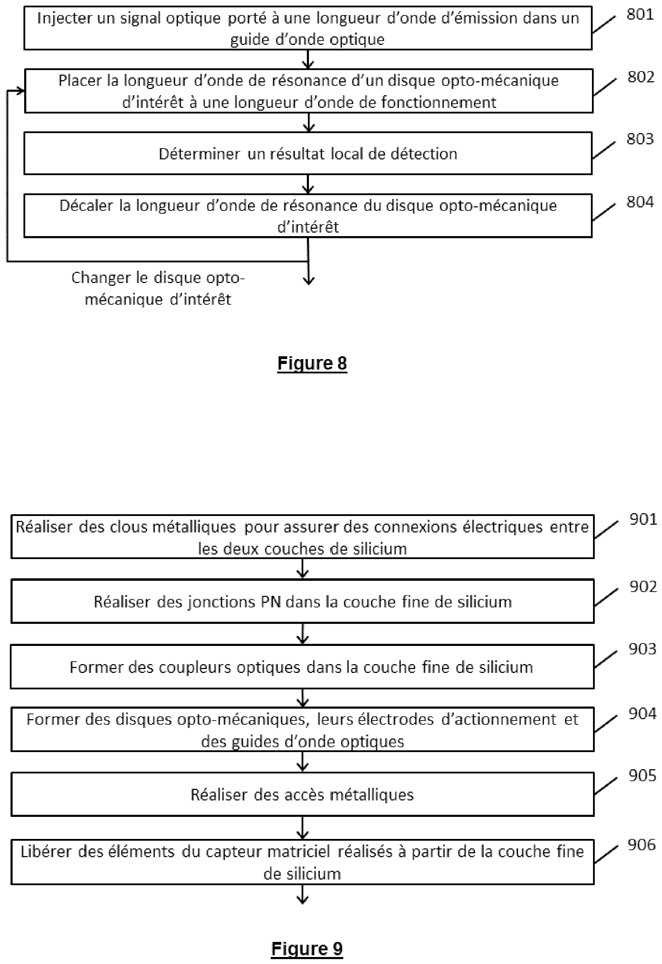

La figure 8 représente un procédé de détection d'espèces chimiques ou biologiques utilisant plusieurs disques opto-mécaniques 201 optiquement couplés avec un guide d'onde optique 202, selon des modes de réalisation de l'invention. Le procédé de détection met en oeuvre les étapes 801 à 804.

A l'étape 801, un signal optique porté à une longueur d'onde d'émission est injecté dans le guide d'onde optique 202, le signal optique n'étant modulé par aucun disque opto-mécanique 201 de la ligne. Une telle configuration peut être obtenue en décalant la longueur d'onde de résonance d'un ou de plusieurs disques 201 et/ou en ajustant la longueur d'onde d'émission de la source laser 101 générant le signal optique.

A l'étape 802, la longueur d'onde de résonance d'un disque opto-mécanique d'intérêt 201 est placée, pendant une fenêtre temporelle de lecture du disque d'intérêt 201, à une longueur d'onde de fonctionnement permettant la modulation du signal optique par le disque opto-mécanique d'intérêt 201, ce qui fournit un signal optique modulé.

A l'étape 803, un résultat local de détection est déterminé à partir du signal optique modulé. Par exemple, le résultat local de détection peut être déterminé en comparant à une fréquence de référence F_{réf} la fréquence de modulation Fₛₒᵣₜᵢₑ d'un signal électrique de détection obtenu en convertissant dans le domaine électrique le signal optique modulé.

A l'étape 804, la longueur d'onde de résonance du disque opto-mécanique d'intérêt 201 est décalée, après l'écoulement de la fenêtre temporelle de lecture du disque d'intérêt 201, de manière à ce que le signal optique ne soit pas modulé par le disque opto-mécanique d'intérêt 201.

Les étapes 802 à 804 peuvent être réitérées pour chacun des disques opto-mécaniques 201, ce qui fournit une pluralité de résultats locaux de détection.

La figure 9 représente un procédé de fabrication d'un capteur matriciel 200, selon des modes de réalisation de l'invention. Le procédé de fabrication utilise une plaque de silicium sur isolant (en anglais :SOI ou Silicon On Insulator) comprenant un empilement d'une couche fine de silicium (Si) d'environ 220 nm d'épaisseur sur une couche d'isolant qui peut être, par exemple et sans limitation, du dioxyde de silicium (SiO₂), l'empilement comprenant en outre une couche épaisse de silicium (Si-Bulk) qui complète l'empilement au niveau de l'autre face de la couche d'isolant. En technologie 200mm, l'épaisseur de cette couche est d'environ 725 µm, mais elle peut être moins épaisse, jusqu'à quelques µm.

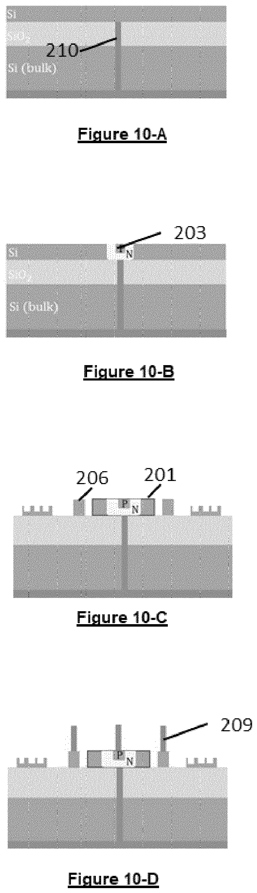

A l'étape 901, des clous métalliques 210, appelés aussi vias, sont réalisés de manière à assurer des connexions électriques entre les deux couches de silicium de la plaque. L'étape 901 consiste en outre à réaliser une électrode commune 205 par métallisation de face arrière de la couche épaisse de silicium. La figure 10-A illustre la sortie de l'étape 901.

A l'étape 902, des jonctions PN 203 sont réalisées dans la couche fine de silicium, chaque jonction PN 203 étant réalisée de manière à être en contact avec un clou métallique. Plus précisément, l'étape 902 peut consister à mettre en oeuvre de manière localisée une implantation de Phosphore et de Bore pour réaliser respectivement les régions dopées N et P de chaque jonction PN 203. La figure 10-B illustre la sortie de l'étape 902.

A l'étape 903, des coupleurs optiques représentant les terminaisons de chaque guide d'onde optique 202 sont formés pour assurer un couplage hors plan avec des fibres optiques. Pour ce faire, l'étape 903 peut consister à mettre en oeuvre des étapes de fabrication comprenant une étape de lithographie, optique ou électronique, opérée au moyen d'un masque dédié sur une couche de résine déposée sur la couche fine de silicium, une étape de développement pour libérer les zones de gravure, et une étape de gravure partielle de la couche fine de silicium pour réaliser une modulation de l'indice optique, la gravure étant de préférence une gravure sèche. L'épaisseur de la gravure, mesurée selon une direction perpendiculaire au plan d'empilement de couches semi-conductrices, peut représenter environ un tiers de l'épaisseur de la couche fine de silicium (environ 70 nm pour une couche fine de silicium d'environ 220 nm).

A l'étape 904, des disques opto-mécaniques 201, leurs électrodes d'actionnement 206 et des guides d'onde optiques sont formés. Plus précisément, l'étape 904 peut consister à mettre en oeuvre des étapes de fabrication comprenant une étape de lithographie, optique ou électronique, opérée au moyen d'un masque dédié sur une couche de résine déposée sur la couche fine de silicium, une étape de développement pour libérer les zones de gravure, et une étape de gravure de la couche fine de silicium, la gravure de la couche fine de silicium étant complète, c'est-à-dire jusqu'à atteindre la couche d'isolant. La figure 10-C illustre la sortie de l'étape 904.

A l'étape 905, des accès métalliques 209 aux disques opto-mécaniques 201 et aux électrodes d'actionnement 206 sont réalisés. Pour ce faire, l'étape 905 peut consister à mettre en oeuvre des étapes de fabrication comprenant une étape de lithographie opérée au moyen d'un masque dédié sur une couche de résine déposée sur la couche fine de silicium, une étape de développement pour libérer l'emplacement des accès métalliques, et une étape de dépôt d'un matériau cible (généralement un métal) qui forme les accès métalliques. La figure 10-D illustre la sortie de l'étape 905.

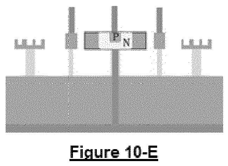

A l'étape 906, des éléments du capteur matriciel 200 réalisés à partir de la couche fine de silicium sont libérés, c'est-à-dire deviennent suspendus au moyen de ce qui reste de la couche d'isolant. De tels éléments libérés comprennent les disques opto-mécaniques 201 et les guides d'onde optiques 202. La libération peut être réalisée au moyen d'une gravure chimique en utilisant l'acide fluorhydrique à l'état gazeux. La figure 10-E illustre la sortie de l'étape 906.

Dans le cadre défini par les revendications annexées, des variantes sont possibles. Par exemple :
- Dans des modes de réalisation de l'invention, l'agencement bidirectionnel de disques opto-mécaniques 201 dans le capteur matriciel 200 peut être géométriquement irrégulier, c'est-à-dire que le nombre de disques 201 et la distance séparant deux disques 201 voisins peuvent varier d'une ligne à une autre et/ou d'une colonne à une autre ;
- Dans d'autres modes de réalisation de l'invention, la source laser 101 mise en oeuvre dans l'unité d'émission 100 peut être de type multi-mode. Dans ce cas, le signal optique généré par la source laser 101 est porté à plusieurs longueurs d'onde d'émission. Dans de tels modes de réalisation de l'invention, le module optique de répartition 102 mis en oeuvre dans l'unité d'émission 100 peut être de type AWG (Arrayed Waveguide Grating), l'AWG étant configuré pour injecter dans chaque guide d'onde optique 202 du capteur matriciel 200 un signal optique filtré porté à une longueur d'onde d'émission ;
- Dans des modes de réalisation de l'invention où la source laser 101 mise en oeuvre dans l'unité d'émission 100 est accordable en longueur d'onde, le circuit de contrôle 300 peut être configuré pour ajuster, préalablement aux fenêtres temporelles de lecture des disques 201 d'une ligne d'intérêt, la longueur d'onde d'émission à laquelle est porté le signal optique injecté dans le guide d'onde optique 202 de la ligne d'intérêt et pour décaler la longueur d'onde de résonance d'un ou de plusieurs disques 201 de la ligne d'intérêt de manière à ce que le signal optique ne soit modulé par aucun disque 201 de la ligne d'intérêt ;
- Dans d'autres modes de réalisation de l'invention, le capteur matriciel 200 peut être réalisé sur une plateforme photonique d'intégration telle que la plateforme silicium. Les avantages d'une telle réalisation en photonique intégré comprennent la miniaturisation, la compacité, ainsi que la faible consommation d'énergie et le faible coût de fabrication.

### REFERENCES

[1] « A Température Controller IC for Maximizing Si Micro-Ring Modulator Optical Modulation Amplitude » Min-Hyeong Kim and al., JOURNAL OF LIGHTWAVE TECHONOLOGY, 2019

## Revendications

1. Dispositif de détection (10) configuré pour détecter des espèces chimiques ou biologiques dans un environnement donné, le dispositif de détection (10) comprenant :
- un capteur matriciel (200) comprenant :
∘ une pluralité de guides d'onde optiques (202), et une pluralité d'électrodes d'actionnement (206) ;
∘ un ensemble de disques opto-mécaniques (201) agencés en lignes et en colonnes, les disques (201) d'une même ligne étant couplés optiquement avec un même guide d'onde optique (202), chaque disque opto-mécanique (201) étant optiquement et mécaniquement résonant et étant apte à se lier à des espèces de l'environnement, les électrodes d'actionnement (206) étant configurés pour assurer la résonance mécanique des disques opto-mécaniques (201) ; et
- une unité d'émission (100) comprenant au moins une source laser (101) configurée pour générer un signal optique porté à une longueur d'onde d'émission, l'unité d'émission (100) étant en outre configurée pour injecter le signal optique dans les guides d'onde optiques (202) du capteur matriciel (200) ;
**caractérisé en ce que** le capteur matriciel comprend également :
∘ une pluralité d'électrodes de polarisation (204) ; et
∘ une pluralité de jonctions PN (203), chaque jonction PN (203) étant associée à un disque opto-mécanique (201), les jonctions PN (203) d'une même colonne étant électriquement connectées à une même électrode de polarisation (204), chaque jonction PN (203) étant configurée pour bloquer la circulation à travers le disque opto-mécanique (201) correspondant d'un courant électrique parasite provenant d'une source autre que l'électrode de polarisation (204) à laquelle la jonction PN (203) est connectée ;
et **en ce que** le le dispositif comprend aussi :
- un circuit de contrôle (300) configuré pour polariser en direct, pendant une fenêtre temporelle de lecture d'un disque d'intérêt (201), la jonction PN (203) du disque d'intérêt (201) de manière à venir placer, par effet thermo-optique, sa longueur d'onde de résonance à une longueur d'onde de fonctionnement, ladite longueur d'onde de fonctionnement étant choisie de manière à ce que l'intensité du signal optique se propageant dans le guide d'onde optique (202) associé au disque d'intérêt (201) soit modulée par ledit disque d'intérêt (201), ce qui fournit un signal optique modulé ; et
- un circuit de lecture (400) configuré pour déterminer à partir du signal optique modulé un résultat local de détection, pendant la fenêtre temporelle de lecture du disque d'intérêt (201).

2. Dispositif de détection (10) selon la revendication 1 dans lequel l'unité d'émission (100) comprend une seule source laser (101), l'unité d'émission (100) comprenant en outre un module optique de répartition (102) configuré pour injecter simultanément le signal optique généré par la source laser (101) dans un ou dans plusieurs guides d'onde optiques (202) du capteur matriciel (200).

3. Dispositif de détection (10) selon la revendication 1 dans lequel l'unité d'émission (100) comprend une pluralité de sources laser (101), le nombre de sources laser (101) étant égal au nombre de guides d'onde optiques (202) dans le capteur matriciel (200), l'unité d'émission (100) étant configurée pour injecter le signal optique généré par chaque source laser (101) dans un guide d'onde optique (202) correspondant dans le capteur matriciel (200).

4. Dispositif de détection (10) selon l'une quelconque des revendications précédentes dans lequel le circuit de contrôle (300) est configuré pour décaler, préalablement aux fenêtres temporelles de lecture des disques (201) d'une ligne d'intérêt, la longueur d'onde de résonance d'un ou de plusieurs disques (201) appartenant à la ligne d'intérêt de manière à ce que le signal optique se propageant dans le guide d'onde optique (202) associé à la ligne d'intérêt ne soit modulée par aucun disque.

5. Dispositif de détection (10) selon l'une quelconque des revendications 1 à 3 dans lequel les sources laser (101) de l'unité d'émission (100) sont accordables en longueur d'onde, le circuit de contrôle (300) étant en outre configuré pour déterminer la longueur d'onde d'émission de chacune des sources laser (101).

6. Dispositif de détection (10) selon la revendication 5 dans lequel le circuit de contrôle (300) est configuré pour ajuster, préalablement aux fenêtres temporelles de lecture des disques (201) d'une ligne d'intérêt, la longueur d'onde d'émission de la source laser (101) associée à la ligne d'intérêt de manière à ce que le signal optique se propageant dans le guide d'onde optique (202) associé à la ligne d'intérêt ne soit modulée par aucun disque.

7. Dispositif de détection (10) selon l'une quelconque des revendications précédentes dans lequel au moins une jonction PN (203) d'un disque opto-mécanique (201) est agencée dans une région centrale du disque opto-mécanique (201) correspondant.

8. Dispositif de détection (10) selon l'une quelconque des revendications précédentes dans lequel chaque disque opto-mécanique (201) est suspendu au moyen d'un support (207) posé sur un substrat commun (208).

9. Dispositif de détection (10) selon la revendication 8 dans lequel au moins une jonction PN (203) d'un disque opto-mécanique (201) est agencée dans la région du substrat commun (208) sur laquelle est posé le support (207) du disque opto-mécanique (201).

10. Dispositif de détection (10) selon l'une quelconque des revendications précédentes dans lequel le circuit de contrôle (300) comprend une pluralité de sources de tension électrique réglables (301), chaque source de tension électrique réglable (301) étant configurée pour appliquer une différence de potentiel électrique entre une électrode de polarisation (204) et une électrode commune (205) de manière à polariser en direct les jonctions PN (203) associées à l'électrode de polarisation (204).

11. Dispositif de détection (10) selon l'une quelconque des revendications précédentes dans lequel le circuit de lecture (400) comprend au moins une unité de lecture (401), l'unité de lecture (401) comprenant un photo-détecteur (4011) configuré pour convertir le signal optique modulé en un signal électrique de détection, l'unité de lecture (401) comprenant en outre un module de traitement (4012) configuré, pendant la fenêtre temporelle de lecture du disque d'intérêt (201), pour :
- déterminer une fréquence de modulation (Fₛₒᵣₜᵢₑ) du signal électrique de détection ;
- calculer une différence de fréquences (ΔF) entre la fréquence de modulation (Fₛₒᵣₜᵢₑ) et une fréquence de référence (F_{réf}) ;
- déterminer le résultat local de détection à partir de la différence de fréquences (ΔF).

12. Dispositif de détection (10) selon l'une quelconque des revendications précédentes dans lequel le dispositif de détection (10) est configuré pour réaliser une lecture séquentielle des lignes du capteur matriciel (200), ce qui fournit pour chaque ligne du capteur matriciel (200) une pluralité de résultats locaux de détection, le dispositif de détection (10) étant en outre configuré pour déterminer un résultat global de détection à partir des pluralités de résultats locaux de détection.

13. Procédé de détection d'espèces chimiques ou biologiques utilisant un dispositif selon la revendication 1, le procédé de détection comprenant les étapes consistant à :
a. injecter (801) dans le guide d'onde optique (202) du dispositif un signal optique porté à une longueur d'onde d'émission, le signal optique n'étant modulé par aucun disque opto-mécanique (201) ;
b. placer (802), pendant une fenêtre temporelle de lecture d'un disque d'intérêt (201) du dispositif, la longueur d'onde de résonance du disque d'intérêt (201) à une longueur d'onde de fonctionnement permettant la modulation du signal optique par le disque opto-mécanique d'intérêt (201), ce qui fournit un signal optique modulé ;
c. déterminer (803) un résultat local de détection à partir du signal optique modulé ;
d. décaler (804), après l'écoulement de la fenêtre temporelle de lecture du disque d'intérêt (201), la longueur d'onde de résonance du disque opto-mécanique d'intérêt (201) de manière à ce que le signal optique ne soit pas modulé par le disque opto-mécanique d'intérêt (201) ;
les étapes b. à d. étant réitérées pour chacun des disques opto-mécaniques (201), ce qui fournit une pluralité de résultats locaux de détection.

14. Procédé de fabrication d'un capteur matriciel (200) d'un dispositif selon la revendication 1 utilisant une plaque de matériaux semi-conducteurs comprenant un empilement d'une couche épaisse de silicium, d'une couche d'isolant et d'une couche fine de silicium, le procédé de fabrication comprenant les étapes suivantes :
- réaliser (901) des clous métalliques (210) de manière à assurer des connexions électriques entre les deux couches de silicium de la plaque ;
- réaliser (902) des jonctions PN (203) dans la couche fine de silicium, chaque jonction PN étant en contact avec un clou métallique ;
- former (903) des coupleurs optiques dans la couche fine de silicium ;
- former (904) des disques opto-mécaniques (201), leurs électrodes d'actionnement (206) et des guides d'onde optiques (202) ;
- réaliser (905) des accès métalliques aux disques opto-mécaniques (201) et aux électrodes d'actionnement (206) ;
- libérer (906) des éléments du capteur matriciel (200) réalisés à partir de la couche fine de silicium.

## Patentansprüche

1. Nachweisvorrichtung (10), konfiguriert zum Nachweisen chemischer oder biologischer Spezies in einer gegebenen Umgebung, wobei die Nachweisvorrichtung (10) Folgendes umfasst:
- einen Matrixsensor (200), der Folgendes umfasst:
o eine Vielzahl von Lichtwellenleitern (202) und eine Vielzahl von Betätigungselektroden (206);
o einen Satz optomechanischer Scheiben (201), die in Zeilen und Spalten angeordnet sind, wobei die Scheiben (201) einer selben Zeile optisch mit einem selben Lichtwellenleiter (202) gekoppelt sind, wobei jede optomechanische Scheibe (201) optisch und mechanisch resonant ist und sich an Spezies der Umgebung binden kann, wobei die Betätigungselektroden (206) zum Sicherstellen der mechanischen Resonanz der optomechanischen Scheiben (201) konfiguriert sind; und
- eine Emissionseinheit (100), die mindestens eine Laserquelle (101) umfasst, die zum Erzeugen eines mit einer Emissionswellenlänge getragenen optischen Signals konfiguriert ist, wobei die Emissionseinheit (100) ferner zum Injizieren des optischen Signals in die Lichtwellenleiter (202) des Matrixsensors (200) konfiguriert ist;
**dadurch gekennzeichnet, dass** der Matrixsensor auch Folgendes umfasst:
o eine Vielzahl von Vorspannungselektroden (204); und
o eine Vielzahl von PN-Übergängen (203), wobei jeder PN-Übergang (203) einer optomechanischen Scheibe (201) zugeordnet ist, wobei die PN-Übergänge (203) einer selben Spalte elektrisch mit einer selben Vorspannungselektrode (204) verbunden sind, wobei jeder PN-Übergang (203) zum Blockieren des Flusses eines von einer anderen Quelle als der Vorspannungselektrode (204), mit der der PN-Übergang (203) verbunden ist, stammenden parasitären elektrischen Stroms durch die entsprechende optomechanische Scheibe (201) konfiguriert ist;
und dadurch, dass die Vorrichtung auch Folgendes umfasst:
- eine Steuerschaltung (300), die zum Vorspannen, während eines Zeitfensters des Lesens einer Scheibe von Interesse (201), des PN-Übergang (203) der Scheibe von Interesse (201) in Durchlassrichtung konfiguriert ist, um über einen thermooptischen Effekt ihre Resonanzwellenlänge auf eine Arbeitswellenlänge zu setzen, wobei die Arbeitswellenlänge so gewählt wird, dass die Stärke des optischen Signals, das sich durch den der Scheibe von Interesse (201) zugeordneten Lichtwellenleiter (202) ausbreitet, durch die Scheibe von Interesse (201) moduliert wird, sodass ein moduliertes optisches Signal geliefert wird; und
- eine Leseschaltung (400), die zum Bestimmen, auf der Basis des modulierten optischen Signals, eines lokalen Nachweisergebnisses während des Zeitfensters des Lesens der Scheibe von Interesse (201) konfiguriert ist.

2. Nachweisvorrichtung (10) nach Anspruch 1, wobei die Emissionseinheit (100) eine einzelne Laserquelle (101) umfasst, wobei die Emissionseinheit (100) ferner ein optisches Verteilungsmodul (102) umfasst, das zum gleichzeitigen Injizieren des von der Laserquelle (101) erzeugten optischen Signals in einen oder in mehrere Lichtwellenleiter (202) des Matrixsensors (200) konfiguriert ist.

3. Nachweisvorrichtung (10) nach Anspruch 1, wobei die Emissionseinheit (100) eine Vielzahl von Laserquellen (101) umfasst, wobei die Anzahl von Laserquellen (101) gleich der Anzahl von Lichtwellenleitern (202) in dem Matrixsensor (200) ist, wobei die Emissionseinheit (100) zum Injizieren des von jeder Laserquelle (101) erzeugten optischen Signals in einen entsprechenden Lichtwellenleiter (202) in dem Matrixsensor (200) konfiguriert ist.

4. Nachweisvorrichtung (10) nach einem der vorhergehenden Ansprüche, wobei die Steuerschaltung (300) zum Verschieben, vor den Zeitfenstern des Lesens der Scheiben (201) einer Zeile von Interesse, der Resonanzwellenlänge einer oder mehrerer zu der Zeile von Interesse gehörenden Scheiben (201) konfiguriert ist, sodass das optische Signal, das sich durch den der Zeile von Interesse zugeordneten Lichtwellenleiter (202) ausbreitet, von keiner Scheibe moduliert wird.

5. Nachweisvorrichtung (10) nach einem der Ansprüche 1 bis 3, wobei die Laserquellen (101) der Emissionseinheit (100) wellenlängenabstimmbar sind, wobei die Steuerschaltung (300) ferner zum Bestimmen der Emissionswellenlänge jeder der Laserquellen (101) konfiguriert ist.

6. Nachweisvorrichtung (10) nach Anspruch 5, wobei die Steuerschaltung (300) zum Justieren, vor den Zeitfenstern des Lesens der Scheiben (201) einer Zeile von Interesse, der Emissionswellenlänge der der Zeile von Interesse zugeordneten Laserquelle (101) konfiguriert ist, sodass das optische Signal, das sich durch den der Zeile von Interesse zugeordneten Lichtwellenleiter (202) ausbreitet, von keiner Scheibe moduliert wird.

7. Nachweisvorrichtung (10) nach einem der vorhergehenden Ansprüche, wobei mindestens ein PN-Übergang (203) einer optomechanischen Scheibe (201) in einer zentralen Region der entsprechenden optomechanischen Scheibe (201) angeordnet ist.

8. Nachweisvorrichtung (10) nach einem der vorhergehenden Ansprüche, wobei jede optomechanische Scheibe (201) mittels eines auf einem gemeinsamen Substrat (208) platzierten Trägers (207) aufgehängt ist.

9. Nachweisvorrichtung (10) nach Anspruch 8, wobei mindestens ein PN-Übergang (203) einer optomechanischen Scheibe (201) in der Region des gemeinsamen Substrats (208) angeordnet ist, auf dem der Träger (207) der optomechanischen Scheibe (201) platziert ist.

10. Nachweisvorrichtung (10) nach einem der vorhergehenden Ansprüche, wobei die Steuerschaltung (300) eine Vielzahl von regulierbaren elektrischen Spannungsquellen (301) umfasst, wobei jede regulierbare elektrische Spannungsquelle (301) zum Anlegen einer elektrischen Potentialdifferenz zwischen einer Vorspannungselektrode (204) und einer gemeinsamen Elektrode (205) konfiguriert ist, um die der Vorspannungselektrode (204) zugeordneten PN-Übergänge (203) in Durchlassrichtung vorzuspannen.

11. Nachweisvorrichtung (10) nach einem der vorhergehenden Ansprüche, wobei die Leseschaltung (400) mindestens eine Leseeinheit (401) umfasst, wobei die Leseeinheit (401) einen Photodetektor (4011) umfasst, der zum Umwandeln des modulierten optischen Signals in ein elektrisches Nachweissignal konfiguriert ist, wobei die Leseeinheit (401) ferner ein Verarbeitungsmodul (4012) umfasst, das während des Zeitfensters des Lesens der Scheibe von Interesse (201) zu Folgendem konfiguriert ist:
- Bestimmen einer Modulationsfrequenz (Fₛₒᵣₜᵢₑ) des elektrischen Nachweissignals;
- Berechnen einer Frequenzdifferenz (ΔF) zwischen der Modulationsfrequenz (Fₛₒᵣₜᵢₑ) und einer Referenzfrequenz (F_{réf});
- Bestimmen des lokalen Nachweisergebnisses auf der Basis der Frequenzdifferenz (ΔF).

12. Nachweisvorrichtung (10) nach einem der vorhergehenden Ansprüche, wobei die Nachweisvorrichtung (10) zum Durchführen eines sequentiellen Lesens der Zeilen des Matrixsensors (200) konfiguriert ist, sodass für jede Zeile des Matrixsensors (200) eine Vielzahl von lokalen Nachweisergebnissen geliefert wird, wobei die Nachweisvorrichtung (10) ferner zum Bestimmen eines globalen Nachweisergebnisses auf der Basis der Vielzahlen von lokalen Nachweisergebnissen konfiguriert ist.

13. Verfahren zum Nachweisen chemischer oder biologischer Spezies unter Verwendung einer Vorrichtung nach Anspruch 1, wobei das Nachweisverfahren die folgenden Schritte umfasst:
a. Injizieren (801) eines mit einer Emissionswellenlänge getragenen optischen Signals in den Lichtwellenleiter (202) der Vorrichtung, wobei das optische Signal durch keine optomechanische Scheibe (201) moduliert wird;
b. Setzen (802), während eines Zeitfensters des Lesens einer Scheibe von Interesse (201) der Vorrichtung, der Resonanzwellenlänge der Scheibe von Interesse (201) auf eine Arbeitswellenlänge, die die Modulation des optischen Signals durch die optomechanische Scheibe von Interesse (201) zulässt, sodass ein moduliertes optisches Signal geliefert wird;
c. Bestimmen (803) eines lokalen Nachweisergebnisses auf der Basis des modulierten optischen Signals;
d. Verschieben (804), nach dem Ablauf des Zeitfensters des Lesens der Scheibe von Interesse (201), der Resonanzwellenlänge der optomechanischen Scheibe von Interesse (201), sodass das optische Signal nicht durch die optomechanische Scheibe von Interesse (201) moduliert wird;
wobei die Schritte b. bis d. für jede der optomechanischen Scheiben (201) wiederholt werden, sodass eine Vielzahl von lokalen Nachweisergebnissen geliefert wird.

14. Verfahren zur Herstellung eines Matrixsensors (200) einer Vorrichtung nach Anspruch 1 unter Verwendung eines Wafers aus Halbleitermaterialien, der einen Stapel aus einer dicken Siliziumschicht, aus einer isolierenden Schicht und aus einer dünnen Siliziumschicht umfasst, wobei das Herstellungsverfahren die folgenden Schritte umfasst:
- Herstellen (901) von Metallnägeln (210), um elektrische Verbindungen zwischen den beiden Siliziumschichten des Wafers herzustellen;
- Herstellen (902) von PN-Übergängen (203) in der dünnen Siliziumschicht, wobei jeder PN-Übergang in Kontakt mit einem Metallnagel ist;
- Ausbilden (903) von optischen Kopplern in der dünnen Siliziumschicht;
- Ausbilden (904) von optomechanischen Scheiben (201), deren Betätigungselektroden (206) und Lichtwellenleitern (202);
- Herstellen (905) von Metallzugängen zu den optomechanischen Scheiben (201) und zu den Betätigungselektroden (206);
- Freisetzen (906) von Elementen des Matrixsensors (200), die aus der dünnen Siliziumschicht hergestellt sind.

## Claims

1. A detecting device (10) configured to detect chemical or biological species in a given environment, the detecting device (10) comprising:
- a matrix-array sensor (200) comprising:
∘ a plurality of optical waveguides (202), and a plurality of actuating electrodes (206);
∘ a set of opto-mechanical discs (201) arranged in rows and columns, the discs (201) of a given row being optically coupled to the same optical waveguide (202), each opto-mechanical disc (201) being optically and mechanically resonant and being able to bind to species of the environment, the actuating electrodes (206) being configured to ensure mechanical resonance of the opto-mechanical discs (201); and
- an emitting unit (100) comprising at least one laser source (101) configured to generate an optical signal carried at an emission wavelength, the emitting unit (100) being further configured to inject the optical signal into the optical waveguides (202) of the matrix-array sensor (200);
**characterised in that** the matrix-array sensor also comprises:
- a plurality of biasing electrodes (204); and
- a plurality of PN junctions (203), each PN junction (203) being associated with one opto-mechanical disc (201), the PN junctions (203) of a given column being electrically connected to the same biasing electrode (204), each PN junction (203) being configured to block the flow through the corresponding opto-mechanical disc (201) of a parasitic electrical current originating from a source other than the biasing electrode (204) to which the PN junction (203) is connected;
and **in that** the device also comprises:
- a control circuit (300) configured to forward bias, during a time window of read-out of a disc of interest (201), the PN junction (203) of the disc of interest (201) so as to place, via a thermo-optical effect, its resonant wavelength at a working wavelength, said working wavelength being chosen so that the strength of the optical signal propagating through the optical waveguide (202) associated with the disc of interest (201) is modulated by said disc of interest (201), thus delivering a modulated optical signal; and
- a read-out circuit (400) configured to determine, on the basis of the modulated optical signal, a local detection result during the time window of read-out of the disc of interest (201).

2. The detecting device (10) according to claim 1, wherein the emitting unit (100) comprises a single laser source (101), the emitting unit (100) further comprising a distributing optical module (102) configured to simultaneously inject the optical signal generated by the laser source (101) into one or more optical waveguides (202) of the matrix-array sensor (200).

3. The detecting device (10) according to claim 1, wherein the emitting unit (100) comprises a plurality of laser sources (101), the number of laser sources (101) being equal to the number of optical waveguides (202) in the matrix-array sensor (200), the emitting unit (100) being configured to inject the optical signal generated by each laser source (101) into a corresponding optical waveguide (202) in the matrix-array sensor (200).

4. The detecting device (10) according to any one of the preceding claims, wherein the control circuit (300) is configured to shift, prior to the time windows of read-out of the discs (201) of a row of interest, the resonant wavelength of one or more discs (201) belonging to the row of interest so that the optical signal propagating through the optical waveguide (202) associated with the row of interest is not modulated by any disc.

5. The detecting device (10) according to any one of claims 1 to 3, wherein the laser sources (101) of the emitting unit (100) are wavelength-tunable, the control circuit (300) being further configured to determine the emission wavelength of each of the laser sources (101).

6. The detecting device (10) according to claim 5, wherein the control circuit (300) is configured to adjust, prior to the time windows of read-out of the discs (201) of a row of interest, the emission wavelength of the laser source (101) associated with the row of interest so that the optical signal propagating through the optical waveguide (202) associated with the row of interest is not modulated by any disc.

7. The detecting device (10) according to any one of the preceding claims, wherein at least one PN junction (203) of an opto-mechanical disc (201) is arranged in a central region of the corresponding opto-mechanical disc (201).

8. The detecting device (10) according to any one of the preceding claims, wherein each opto-mechanical disc (201) is suspended by means of a holder (207) placed on a common substrate (208).

9. The detecting device (10) according to claim 8, wherein at least one PN junction (203) of an opto-mechanical disc (201) is arranged in the region of the common substrate (208) on which the holder (207) of the opto-mechanical disc (201) is placed.

10. The detecting device (10) according to any one of the preceding claims, wherein the control circuit (300) comprises a plurality of adjustable electrical voltage sources (301), each adjustable electrical voltage source (301) being configured to apply an electrical potential difference between a biasing electrode (204) and a common electrode (205) so as to forward bias the PN junctions (203) associated with the biasing electrode (204).

11. The detecting device (10) according to any one of the preceding claims, wherein the read-out circuit (400) comprises at least one read-out unit (401), the read-out unit (401) comprising a photodetector (4011) configured to convert the modulated optical signal into an electrical detection signal, the read-out unit (401) further comprising a processing module (4012) configured, during the time window of read-out of the disc of interest (201), to:
- determine a modulation frequency (Fₛₒᵣₜᵢₑ) of the electrical detection signal;
- compute a frequency difference (ΔF) between the modulation frequency (Fₛₒᵣₜᵢₑ) and a reference frequency (F_{réf});
- determine the local detection result on the basis of the frequency difference (ΔF).

12. The detecting device (10) according to any one of the preceding claims, wherein the detecting device (10) is configured to carry out a sequential read-out of the rows of the matrix-array sensor (200), which delivers, for each row of the matrix-array sensor (200), a plurality of local detection results, the detecting device (10) being further configured to determine a global detection result on the basis of the pluralities of local detection results.

13. A method for detecting chemical or biological species using a device according to claim 1, the detecting method comprising the steps of:
a. injecting (801), into the optical waveguide (202) of the device, an optical signal carried at an emission wavelength, wherein the optical signal is not modulated by any opto-mechanical disc (201);
b. placing (802), during a time window of read-out of a disc of interest (201) of the device, the resonant wavelength of the disc of interest (201) at a working wavelength allowing modulation of the optical signal by the opto-mechanical disc of interest (201), thus delivering a modulated optical signal;
c. determining (803) a local detection result on the basis of the modulated optical signal;
d. shifting (804), after the time window of read-out of the disc of interest (201) has passed, the resonant wavelength of the opto-mechanical disc of interest (201) so that the optical signal is not modulated by the opto-mechanical disc of interest (201);
steps b. to d. being reiterated for each of the opto-mechanical discs (201), thus delivering a plurality of local detection results.

14. A method of manufacturing a matrix-array sensor (200) of a device according to claim 1 using a wafer of semiconductor materials comprising a stack of a thick silicon layer, of an insulating layer and of a thin silicon layer, the manufacturing method comprising the following steps:
- producing (901) metal nails (210) so as to ensure electrical connections between the two silicon layers of the wafer;
- producing (902) PN junctions (203) in the thin silicon layer, each PN junction making contact with one metal nail;
- forming (903) optical couplers in the thin silicon layer;
- forming (904) opto-mechanical discs (201), the actuating electrodes (206) thereof and optical waveguides (202);
- producing (905) metal accesses to the opto-mechanical discs (201) and to the actuating electrodes (206);
- releasing (906) elements of the matrix-array sensor (200), which are produced from the thin silicon layer.
